# EUROPÄISCHE PATENTSCHRIFT

(11) **EP 1 338 247 B1**
(45) Veröffentlichungstag und Bekanntmachung des Hinweises auf die Patenterteilung: **14.04.2004**
(21) Anmeldenummer: 02003286.8
(22) Anmeldetag: 22.02.2002
(51) Int. Cl.: A61B 6/00, A61B 19/00

(54) **Höhenreduziertes Kalibrierungsinstrument**
Calibration device with reduced height
Outil de calibration à hauteur réduite

(43) Veröffentlichungstag der Anmeldung: 27.08.2003
(60) Teilanmeldung: 03011087.8
(73) Patentinhaber: BrainLAB AG, 85551 Kirchheim/Heimstetten (DE)
(72) Erfinder: Zeiss, Mario, 85586 Poing (DE)
(74) Vertreter: Schwabe - Sandmair - Marx

(56) Entgegenhaltungen:
- WO-A-00/64367
- DE-A- 19 856 536
- FR-A- 2 631 810
- US-A- 5 772 594
- US-A- 5 951 475
- US-A- 6 118 845

## Beschreibung

Die Erfindung betrifft ein höhenreduziertes Kalibrierungsinstrument zur Navigations-Kalibrierung von Röntgenbilddaten. Im Besonderen betrifft die vorliegende Erfindung das Gebiet der computerunterstützten Chirurgie und der bildunterstützten Chirurgie, bei der dem behandelnden Chirurgen ein medizinisches Navigationssystem mit Bildschirmausgabe zur Verfügung steht, das ihn bei den vorzunehmenden Eingriffen leitet bzw. unterstützt.

Im Rahmen einer solchen medizinischen Navigation kann zusätzlich auf Informationen zurückgegriffen werden, die aus vor Ort erstellten Röntgenbildern ermittelt werden, wobei dabei auf der einen Seite zusätzliche und wichtige anatomische Informationen aus dem Röntgenbild in die Navigations-Bildunterstützung eingebracht werden können und andererseits auch die Möglichkeit besteht, mit Hilfe der Röntgenbilder die Registrierung des Patienten im Navigationssystem zu aktualisieren.

Ein solches System ist beispielsweise aus der EP 1 153 572 A1 bekannt.

Weitere Dokumente, die sich mit dem technischen Hintergrund zur oben genannten "Röntgennavigation" beschäftigen, sind die US-Patente Nr. 5,799,055, Nr. 3,577,160, Nr. 6,118,845, Nr. 5,784,431, Nr. 5,967,982 und Nr. 5,772,594.

Eine solche röntgenbildunterstützte Navigation kann für verschiedene medizinische Eingriffe verwendet werden, beispielsweise für Wirbelsäulenoperationen und im Rahmen der Unfallchirurgie. Hierfür wird sowohl eine geeignete Software im Navigationssystem als auch ein Kalibrierungsinstrument benötigt, um mit einem Röntgengerät, beispielsweise einem C-Bogen, die Navigation auf registrierten/kalibrierten Bilddaten zu ermöglichen.

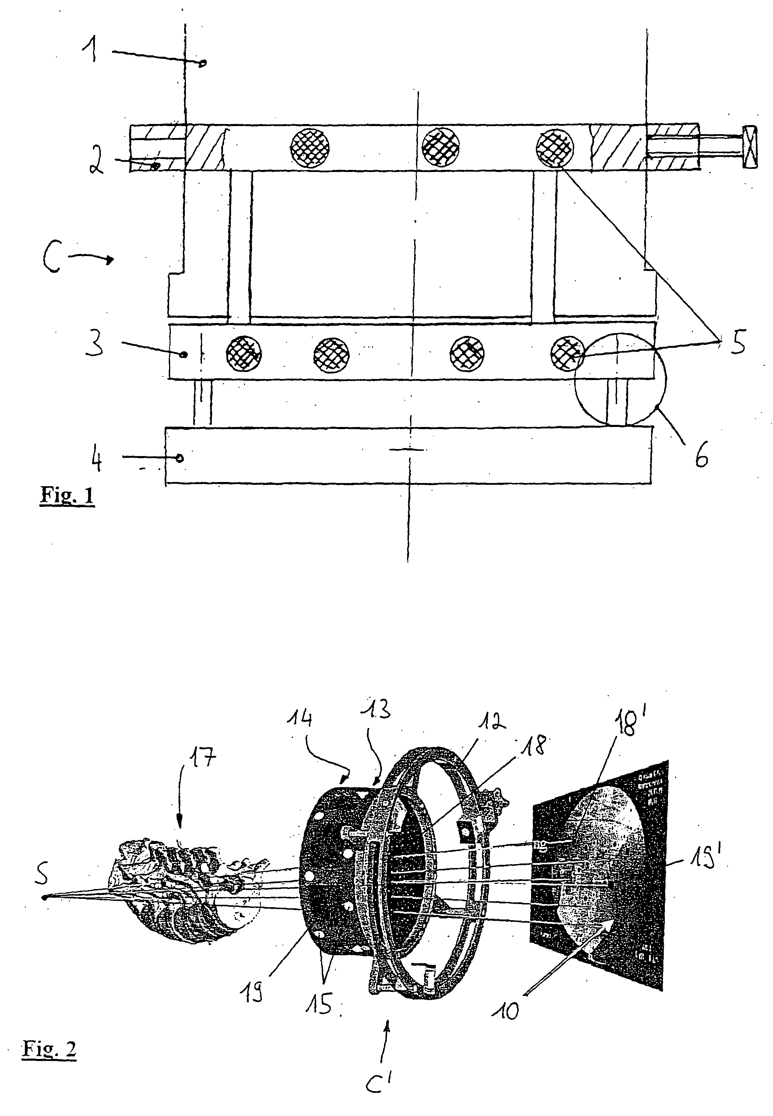

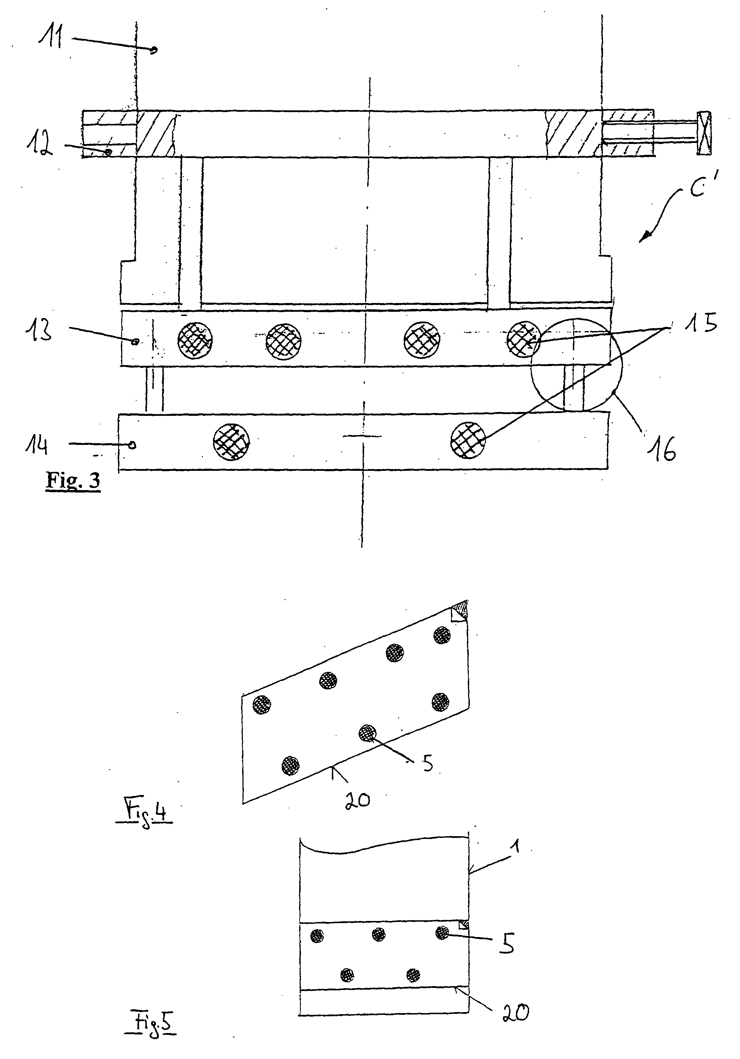

Ein Beispiel für ein herkömmlicherweise verwendetes Kalibrierungsinstrument ist in der perspektivischen Darstellung der Figur 2 zu sehen; einen schematischen Aufriss dieses Instruments stellt die Figur 3 dar. Dieses herkömmliche Kalibrierungsinstrument C' besteht aus einer Fixierungseinrichtung 12, mit Hilfe derer es beispielsweise am Bildaufnehmer eines C-Bogen-Röntgengerätes befestigt werden kann. Dieser Bildaufnehmer (Detektor) ist in Figur 3 nur andeutungsweise gezeigt (Bezugszeichen 11). An der Fixierungsvorrichtung 12 sind in einem Abstand nacheinander ein erster Träger 13 sowie ein zweiter Träger 14 angebracht, die Platten umgrenzen, auf denen Lokalisationsinformationen aufgebracht wurden, nämlich beispielsweise Strukturen in den beiden Platten wie Wolfram-Kugeln, Linienstrukturen usw., die sowohl für die Bildentzerrung als auch für die Orientierung des Bildes verwendet werden. Je eine solche Lokalisationsstruktur, nämlich jeweils eine Wolfram-Kugel ist aus den in Figur 2 dargestellten Platten mit dem Bezugszeichen 18 (Träger 13) bzw. 19 (Träger 14) gezeigt. Diese Strukturen besitzen eine definierte Anordnung, und auch die Träger bzw. Platten sind in einem definierten Abstand zueinander angeordnet, um die Berechnung einer virtuellen Strahlungsquelle zu ermöglichen, die in Figur 2 mit dem Bezugszeichen S versehen ist. Je größer der Abstand der Ebenen bzw. Träger 13, 14, umso genauer kann die Strahlungsquelle berechnet werden und umso genauer kann auf den registrierten Bilddaten navigiert werden.

In Figur 2 ist gezeigt, wie eine solche Abbildung, nämlich das erzeugte Röntgenbild 10, aussehen könnte. Die Röntgenstrahlen gehen von der virtuellen Strahlenquelle S durch den abzubildenden Körperteil, wobei in Figur 2 ein Teil einer Wirbelsäule 17 gezeigt ist. Danach durchdringen die Röntgenstrahlen die erste und die zweite Platte auf den Trägern 14, 13 des Kalibrierungsinstrumentes C', wodurch letztendlich auf dem Röntgenbild 10 die Abbildung der Wirbelsäule zusammen mit den Abbildungen 18', 19' der Lokalisationsstrukturen vorhanden ist. Da die Vielzahl der Lokalisationsstruktur bei einem bestimmten Anstrahlungswinkel immer genau eine zuordnungsfähige Abbildungsanordnung ergibt, lässt sich aus den Abbildungen dieser Strukturen exakt auf die Lage der virtuellen Strahlungsquelle S zurückschließen. Dadurch erhält man das genaue Wissen um die Orientierung des erstellten Röntgenbildes.

Zusätzlich befinden sich am Kalibrierungsinstrument C' noch Sensoren bzw. Markierungen 15, die LED's, reflektive Marker oder Magnetsensoren sein können und die es einem medizinischen Navigationssystem ermöglichen, mit Hilfe einer Software die Position des Kalibrierungsinstruments C' im Raum festzustellen. Aus den Informationen über die Raumposition des Kalibrierungsinstrumentes sowie über die Orientierung des Röntgenbildes lässt sich nun die Position des Röntgenbildes im Raum selbst feststellen und in die Navigation einbinden. In Figur 3 ist zusätzlich noch mit dem Bezugszeichen 16 die Verbindung zwischen dem ersten Träger 13 und dem zweiten Träger 14 angedeutet, es handelt sich hier um eine starre und feste Verbindung; die beiden Träger 13, 14 sind einteilig miteinander verbunden.

Derzeit besteht bei der Verwendung solcher Kalibrierungsinstrumente im Rahmen der Röntgennavigation das Problem, dass die Höhe des Kalibrierungsinstrumentes aufgrund der zwei, in festem Abstand zueinander angebrachten Träger mit Kalibrierungsinformationen die "lichte Weite" des Röntgengerätes sehr stark einschränkt. Wenn ein C-Bogen verwendet wird, sitzt das Kalibrierungsinstrument zum Beispiel auf dem Bilddetektor und verkürzt den Abstand zwischen diesem und dem Röntgengenerator beträchtlich. Dies ist besonders bei Wirbelsäulenoperationen im lumbalen Bereich aber auch bei Hüftoperationen kritisch, so dass in diesen Fällen häufig auf eine Röntgennavigation verzichtet werden muss.

Es ist die Aufgabe der vorliegenden Erfindung, ein Kalibrierungsinstrument bereitzustellen, welche das obige Problem überwindent; insbesondere soll die Möglichkeit geschaffen werden, die lichte Weite eines Röntgengerätes mit einem Kalibrierungsinstrument ausreichend groß zu gestalten, um den Einsatz der Röntgennavigation auch für bisher ausgeschlossene Fälle zu gestatten.

Diese Aufgabe wird durch ein Kalibrierungsinstrument gemäß Anspruch 1 gelöst. Die Unteransprüche definieren bevorzugte Ausführungsformen der Erfindung.

Ein Verfahren zur Navigations-Kalibrierung von Röntgenbilddaten weist beispielsweise die folgenden Schritte auf:
- mittels eines Röntgengerätes, in dessen Strahlungsweg ein Kalibrierungsinstrument eingebracht ist, das in einem medizinischen Navigationssystem positionell erfasst und verfolgt werden kann, wird eine Röntgenaufnahme ohne Patient erstellt,
- mit Hilfe von Lokalisationsstrukturen, die auf mindestens zwei beabstandeten Trägem des Kalibrierungsinstruments angeordnet sind, und deren Abbildungen auf der ersten Röntgenaufnahme wird die Röntgenaufnahme ohne Patient im Navigationssystem registriert.
- ein Träger mit seinen Lokalisationsstrukturen wird vom Kalibrierungsinstrument abgenommen,
- eine Patienten-Röntgenaufnahme wird erstellt,
- die Kalibrierungsinformationen aus den Abbildungen der Lokalisationsstrukturen des am Instrument verbleibenden Trägers werden für beide Röntgenaufnahmen verglichen und die Kalibrierung wird bei mangelnder Übereinstimmung der Abbildungen korrigiert.

Der besondere Vorteil eines solchen Verfahrens liegt in dem Abnehmen mindestens eines Trägers mit Lokalisationsstrukturen vom Kalibrierungsinstrument und der daraufhin durchgeführten Kompensation des hieraus entstehenden Informationsmangels. Diese Maßnahmen ermöglichen es nämlich, eine Navigations-Kalibrierung für Röntgenbilder auch in Fällen durchzuführen, wo eine sehr große lichte Weite bzw. ein sehr großer Abstand zwischen Röntgengenerator und Röntgendetektor benötigt wird, also beispielsweise bei Wirbelsäulenoperationen im lumbalen Bereich oder auch bei Hüftoperationen. Durch eine geeignete Ausgestaltung des Kalibrierungsinstrumentes und seine geeignete Verwendung kann eine Röntgennavigation auch bei Operationen verwendet werden kann, für die eine solche Unterstützung bisher nicht möglich war.

Die Röntgenaufnahme kann ohne Patient zuerst erstellt werden, um eine Basiskalibrierung des Röntgengerätes durchzuführen. Mit einem solchen, sogenannten "blank shot" mit beiden Trägern des Kalibrierungsinstrumentes kann vor der eigentlichen Bilderstellung mit dem Patientenkörperteil schon die Bildorientierung bzw. die Lage der virtuellen Strahlungsquelle für das verwendete Röntgengerät, das vorzugsweise ein C-Bogen-Röntgengerät ist, ermittelt werden. Hierbei sind keine Hindernisse im Strahlenbereich vorhanden, so dass die Einschränkung der lichten Weite zwischen Röntgengenerator und Röntgendetektor keine Rolle spielt.

Ein solcher "blank shot" kann aber auch dann verwendet werden, wenn der behandelnde Arzt feststellt, dass bei Röntgenaufnahme mit Patient, also bei der Patienten-Röntgenaufnahme, Teile der Lokalisationsstrukturen verdeckt sind, so dass eine eindeutige Bildorientierung nicht mehr ermittelt werden kann. Ein neuer blank shot ermöglicht dann wieder eine genaue Kalibrierung des Systems.

Beim oben beschriebenen Verfahren werden die Kalibrierungsinformationen aus den Abbildungen der Lokalisationsstrukturen des am Instrument verbleibenden Trägers für beide Röntgenaufnahmen (mit und ohne Patient) verglichen, und die Kalibrierung wird bei mangelnder Übereinstimmung der Abbildungen korrigiert. Für eine solche Korrektur bzw. Kompensation bieten sich mehrere Möglichkeiten an.

Erstens kann als Korrektur eine Neukalibrierung mittels einer Röntgenaufnahme ohne Patient, vorzugsweise in derselben Stellung des Röntgengerätes, in welcher die Patienten-Röntgenaufnahme erfolgt ist, durchgeführt werden. Insbesondere C-Bogen-Röntgengeräte haben möglicherweise in verschiedenen Einsatzstellungen unterschiedliche Relativpositionen für Röntgengenerator und Röntgendetektor. Wenn der C-Bogen horizontal ausgerichtet ist, kann dieses Positionsverhältnis möglicherweise anders sein, als bei einem vertikal aufgestellten C-Bogen. Aus diesem Umstand könnten sich Verzerrungen ergeben, die Fehler in der Bestimmung der Orientierung des Röntgenbildes mit sich brächten. Wenn also beispielsweise der blank shot bzw. eine Basiskalibrierung mit vertikalem C-Bogen durchgeführt wurde, kann es vorkommen, dass es bei einem horizontalen Einsatz des C-Bogens die Abbildungen der Lokalisationsstrukturen des am Instrument verbleibenden Trägers bei der Aufnahme mit Patient schon verschoben sind. Gemäß der oben genannten Ausführungsform würde dann eine einfache neue Kalibrierung in derselben Stellung wie bei der Patienten-Röntgenaufnahme wieder ein geeignet kalibriertes System schaffen.

Eine zweite Möglichkeit besteht darin, bei der Korrektur aus der Veränderung der Abbildungsdaten die Änderung der Aufnahmesituation computergestützt zu errechnen, wobei dann für die Aufnahmesituation bei der Patienten-Röntgenaufnahme neue Abbildungsdaten für die Lokalisierungsvorrichtungen des abgenommenen Trägers ermittelt werden. Mit anderen Worten wird aus der Verschiebung der Abbildungen für den festen Träger auch die Verschiebung der Abbildungen für den abgenommenen Träger durch Berechnung ermittelt bzw. rekonstruiert. Bei geeigneter Rechenleistung und Genauigkeit erspart ein solches System alle eventuell umständlichen Neukalibrierungs-Maßnahmen.

Im dritten möglichen Fall wird aus der mangelnden Übereinstimmung ein Abweichungswert ermittelt, und, wenn dieser Abweichungswert eine vorbestimmte Grenze nicht überschreitet, wird die vorhandene Kalibrierung als korrigierte Kalibrierung übernommen. Wenn also festgestellt wird, dass nur eine geringe Abweichung im Rahmen vorgegebener Toleranzen vorliegt, kann für diesen Fall - ohne Probleme befürchten zu müssen - mit der bereits vorgenommenen Kalibrierung (blank shot vorab) weitergearbeitet werden. Die Positionen für die Abbildungen des abgenommenen Trägers werden einfach aus dem blank shot übernommen.

Eine vierte Möglichkeit besteht darin, auf der Basis einer vorab für das Röntgengerät ermittelten Interpolationsregel die korrigierte Kalibrierung aus den Abweichungen zu ermitteln. Hier wird vorteilhaft von der Tatsache Gebrauch gemacht, dass ein bestimmtes Röntgengerät bzw. ein bestimmter C-Bogen in verschiedenen Einsatzstellungen sehr wahrscheinlich immer dieselben Abweichungen zeigt. Diese Abweichungen lassen sich vorab erfassen, und dieses Wissen kann dann verwendet werden, um eine Korrektur bzw. Kompensation in bestimmten Einsatzfällen zu bestimmen. Die Interpolation kann mittels Wertepaar-Listen oder über eine empirisch ermittelte Funktion stattfinden.

Das erfindungsgemäße Kalibrierungsinstrument für die Navigations-Kalibrierung von Röntgenbilddaten weist auf: eine Vorrichtung zum Anordnen des Kalibrierungsinstruments im Strahlungsweg eines Röntgengerätes, mindestens zwei beabstandete Träger, auf denen Lokalisationsstrukturen vorgesehen sind, die auf den Röntgenaufnahmen des Röntgengerätes abgebildet werden, und Markierungen für die Erfassung des Kalibrierungsinstrumentes durch ein medizinisches Navigationssystem. Es ist dadurch gekennzeichnet, dass mindestens einer der Träger vom Kalibrierungsinstrument abnehmbar ist. Durch diese Ausgestaltung des Kalibrierungsinstrumentes, insbesondere durch die Abnehmbarkeit eines der Träger für die Lokalisationsstrukturen wird ein höhenreduziertes Kalibrierungsinstrument geschaffen, das auch für bisher problematische Anwendungen eine ausreichend große lichte Weite aufweist. Die oben für das Verfahren beschriebenen Vorteile gelten naturgemäß auch für das erfindungsgemäße, höhenreduzierte Kalibrierungsinstrument.

Gemäß einer Ausführungsform des Instruments sind die Lokalisationsstrukturen durch Röntgenstrahlung abbildbare Kugel- oder Linienstrukturen, insbesondere z.B.aus Wolfram. Das Kalibrierungsinstrument kann vorteilhafterweise am Bildaufnehmer eines Röntgengerätes, insbesondere eines C-Bogen-Röntgengerätes angeordnet sein.

Mit Vorteil wird der abnehmbare Träger mittels eines Schnellverschlusses an dem nicht abnehmbaren Träger angeordnet, und ein solcher Schnellverschluss kann ein Dreh-Bajonettverschluss oder eine Steckverbindung sein. Wichtig ist dabei, dass die Verbindung eine genaue Positionierung und Repositionierung jedes abnehmbaren Trägers zulässt, damit die Genauigkeit der Kalibrierungsschritte gewährleistet ist.

Die Markierungen für die Erfassung des Kalibrierungsinstrumentes durch ein medizinisches Navigationssystem werden bei einer vorteilhaften Ausführungsform auf dem nicht abnehmbaren Träger bzw. auf der Vorrichtung zum Anordnen des Kalibrierungsinstrumentes im Strahlungsweg des Röntgengerätes angeordnet. Letztere Vorrichtung kann die Befestigungsvorrichtung sein, mit der das Instrument am Bildaufnehmer des Röntgengerätes befestigt wird. Wenn mehrere oder alle Träger abgenommen werden, erweist sich eine weitere Ausführungsform als besonders vorteilhaft. Bei dieser werden die Markierungen auf separaten Unterlagen, z.B. Klebefolien vorgesehen, die am Röntgengerät selbst angebracht werden können, insbesondere am Bildverstärker/Detektor. Bevorzugt wird diese Ausführungsform eingesetzt, wenn eine Vorkalibrierung erfolgt ist, und insbesondere dann, wenn die korrigierte Kalibrierung über Interpolationsregeln ermittelt wird.

Die Erfindung wird im Weiteren auch unter Bezugnahme auf die Figur 1 näher erläutert. In den beiliegenden Zeichnungen zeigen:
- Figur 1: einen Aufriss für ein erfindungsgemäßes, höhenreduziertes Kalibrierungsinstrument;
- Figur 2: eine perspektivische Darstellung der Röntgenbilderstellung mit Hilfe eines Kalibrierungsinstrumentes nach dem Stand der Technik;
- Figur 3: einen schematischen Aufriss für ein Kalibrierungsinstrument nach dem Stand der Technik; und
- Figuren 4 und 5: eine Klebefolie mit passiven Markern und ihre Anbringung an einem Bildaufnehmer.

Das in Figur 1 dargestellte Kalibrierungsinstrument C weist im Wesentlichen dieselben Bauteile auf, wie das oben erläuterte Kalibrierungsinstrument C' gemäß dem Stand der Technik. An dem Bildaufnehmer 1 eines C-Bogen-Röntgengerätes ist das Kalibrierungsinstrument C mit der Fixierungsvorrichtung 2 angebracht, die in fester Verbindung zu dem ersten Träger 3 steht. Auf der Fixierungsvorrichtung 2 und am ersten Träger 3 sind die Markierungen 5 angebracht, mit Hilfe derer das Kalibrierungsinstrument in einem medizinischen Navigationssystem positionell erfasst und verfolgt wird. Mit einer lösbaren Befestigungseinrichtung 6, beispielsweise einem Bajonett-Schnellverschluss oder einem Einsteck-Rastverschluss ist am Träger 3 der Träger 4 befestigt, der ebenso wie der Träger 3 eine Platte umschließt, die Lokalisationsstrukturen, zum Beispiel Wolfram-Kugeln oder Linienstrukturen aufweist.

Der Träger 4 ist vom Träger 3 abnehmbar, und dadurch wird die Höhe des Kalibrierungsinstrumentes, das bei der Patienten-Röntgenaufnahme dann nur noch den Träger 3 aufweist, stark reduziert, und die lichte Weite des Röntgengerätes wird entsprechend größer. Im Gegensatz zu der Darstellung in Figur 3 ist in Figur 1 zu sehen, dass die Markierungen 5 nun nicht mehr an beiden Trägern (13, 14 in Figur 3) angebracht sind, sondern an dem Träger 3 und an der Fixierungseinrichtung 2, die auch während der Patienten-Röntgenaufnahme am Kalibrierungsinstrument verbleiben.

Im Einsatz werden mit dem erfindungsgemäßen Kalibrierungsinstrument die folgenden Tätigkeiten durchgeführt. Zunächst wird ein sogenannter "blank shot" mit dem Röntgengerät ausgeführt, bei dem noch kein Patient im Strahlengang vorhanden ist, und für den ferner beide Träger 3, 4 mit den Lokalisationsstrukturen am Kalibrierungsinstrument angebracht sind. Die Kalibrierungsinformationen mit den Lokalisationsstrukturen aus beiden Ebenen werden gespeichert. Der blank shot kann einmal durchgeführt werden, es können aber auch mehrere blank shots, beispielsweise in Anterior-Posterior-Richtung, lateraler Richtung oder in Schrägrichtungen (X-Aufnahmen) gemacht werden.

Danach wird der Träger 4 von dem Träger 3 über den Schnellverschluss 6 abgenommen, und das Röntgengerät wird über dem Patienten positioniert.

Die zu kalibrierende Patienten-Röntgenaufnahme wird nunmehr erstellt, wobei lediglich diejenigen Lokalisationsstrukturen auf dem Röntgenbild abgebildet werden, die am Träger 3 vorhanden sind. Die fehlenden Informationen aus der zweiten Ebene, also von den Lokalisationsstrukturen des Trägers 4 werden rekonstruiert, und hierfür bestehen mehrere Möglichkeiten, von denen oben vier beschrieben wurden. Wenn keine Abweichungen für die Lokalisationsstrukturen des Trägers 3 ermittelt werden, können die Informationen aus dem blank shot einfach übernommen werden. Bei Abweichungen kann aus der Natur der Abweichung heraus die Sollposition der Lokalisationsstrukturen des abgenommenen Trägers errechnet oder interpoliert werden, oder es wird eine Neu-Kalibrierung mit einem nochmaligen blank shot in gleicher Aufnahmeposition durchgeführt.

In jedem Fall wird eine Plausibilitätsprüfung durch das behandelnde Personal durchgeführt, diese kann rein visuell oder auch computerunterstützt erfolgen und dabei wird festgestellt, ob die Rekonstruktion der Lokalisationsdaten des Trägers 4 sinnvoll sein kann.

Wenn die Plausibilitätsprüfung erfolgreich war, erfolgt dann die Bildkalibrierung, die Registrierung sowie die röntgenunterstützte Navigation, nachdem noch die Genauigkeit überprüft worden ist.

In den Figuren 4 und 5 wird noch dargestellt, wie gemäß einer weiteren Ausführungsform der Erfindung Markierungen, nämlich passive Reflexionsmarker 5 am Bildaufnehmer 1 des Röntgengeräts angebracht werden können. Eine solche Ausführungsform eignet sich insbesondere dann, wenn mehrere oder alle Träger mit den Lokalisationsstrukturen abnehmbar ausgestaltet sind und auch abgenommen werden sollen. Es wird eine Kunststofffolie 20 als separate Unterlage vorgesehen, die auf einer Seite eine bevorzugt wieder lösbare Klebeschicht aufweist und passive Reflexionsmarker 5 trägt. Speziell dann, wenn eine Vorkalibrierung erfolgt ist, kann somit die Anbringung von Positionierungsinformationen (Marker 5 auf Klebefolie 20) direkt am Bildaufnehmer/-verstärker bzw. Detektor erfolgen, ohne dass weitere mechanische oder konstruktive Umsetzungen nötig sind.

Die vorliegende Erfindung ermöglicht somit eine Höhenreduktion des Kalibrierungsinstrumentes, ohne dabei die Genauigkeit für eine Röntgennavigation einzuschränken, die im erheblichen Maße von der Registrierung und damit vom Kalibrierungsinstrument abhängt.

## Patentansprüche

1. Kalibrierungsinstrument für die Navigations-Kalibrierung von Röntgenbilddaten mit einer Vorrichtung (2) zum Anordnen des Kalibrierungsinstruments (C) im Strahlungsweg eines Röntgengerätes, mit mindestens zwei beabstandeten Trägem (3, 4), auf denen Lokalisationsstrukturen (18, 19) vorgesehen sind, die auf den Röntgenaufnahmen des Röntgengeräts abgebildet werden, und mit Markierungen (5) für die Erfassung des Kalibrierungsinstruments (C) durch ein medizinisches Navigationssystem, **dadurch gekennzeichnet, dass** mindestens einer der Träger (4) vom Kalibrierungsinstrument (C) abnehmbar ist.

2. Kalibrierungsinstrument nach Anspruch 1, **dadurch gekennzeichnet, dass** die Lokalisationsstrukturen durch Röntgenstrahlung abbildbare Kugel- oder Linienstrukturen, insbesondere aus Wolfram, sind.

3. Kalibrierungsinstrument nach Anspruch 1 oder 2, **dadurch gekennzeichnet, dass** es am Bildaufnehmer des Röntgengeräts angeordnet ist.

4. Kalibrierungsinstrument nach einem der Ansprüche 1 bis 3, **dadurch gekennzeichnet, dass** der annehmbare Träger (4) mittels eines Schnellverschlusses an dem nicht abnehmbaren Träger (3) angeordnet ist.

5. Kalibrierungsinstrument nach Anspruch 4, **dadurch gekennzeichnet, dass** zur Befestigung des abnehmbaren Trägers (4) ein Dreh-Bajonettverschluss oder eine Steckverbindung verwendet wird.

6. Kalibrierungsinstrument nach einem der Ansprüche 1 bis 5, **dadurch gekennzeichnet, dass** die Markierungen (5) für die Erfassung des Kalibrierungsinstruments (C) durch ein medizinisches Navigationssystem auf dem nicht abnehmbaren Träger (3) bzw. auf der Vorrichtung (2) zum Anordnen des Kalibrierungsinstruments (C) im Strahlungsweg des Röntgengerätes angeordnet sind.

7. Kalibrierungsinstrument nach einem der Ansprüche 1 bis 5, **dadurch gekennzeichnet, dass** die Markierungen (5) auf einer separaten Unterlage, insbesondere auf einer bevorzugt wiederablösbaren Klebefolie (20) angeordnet sind, die am Röntgengerät bzw. am Bildaufnehmer (1) des Röntgengeräts angebracht werden.

## Claims

1. A calibration instrument for navigation-calibrating x-ray image data, comprising: a device (2) for arranging said calibration instrument (C) in the radiation path of an x-ray device; at least two spaced supports (3, 4) on which localisation structures (18, 19) are provided which are imaged on said x-ray images of said x-ray device; and markings (5) for detecting said calibration instrument (C) using a medical navigation system, **characterised in that** at least one of said supports (4) may be removed from said calibration instrument (C).

2. The calibration instrument as set forth in claim 1, **characterised in that** said localisation structures are spherical or line structures, in particular made of tungsten, which may be imaged using x-rays.

3. The calibration instrument as set forth in claim 1 or 2, **characterised in that** it is arranged on the image recorder of said x-ray device.

4. The calibration instrument as set forth in any one of claims 1 to 3, **characterised in that** said removable support (4) is arranged on said non-removable support (3) by means of a quick-release lock.

5. The calibration instrument as set forth in claim 4, **characterised in that** a rotating bayonet lock or a plug-type connection is used to fix said removable support (4).

6. The calibration instrument as set forth in claim 1 or 5, **characterised in that** said markings (5) for detecting said calibration instrument (C) using a medical navigation system are arranged on said non-removable support (3) or on said device (2) for arranging said calibration instrument (C) in the radiation path of said x-ray device.

7. The calibration instrument as set forth in claim 1 or 5, **characterised in that** said markings (5) are arranged on a separate base layer, in particular on a preferably detachable adhesive film (20), which is attached to said x-ray device or to said image recorder (1).

## Revendications

1. Outil de calibrage pour le calibrage de navigation de données d'image radiographique avec un dispositif (2) pour disposer l'outil de calibrage (C) dans le chemin de rayonnement d'un appareil à rayons X, avec au moins deux supports écartés (3, 4), sur lesquels des structures de localisation (18, 19) sont prévues, qui sont reproduites sur les images radiographiques de l'appareil à rayons X, et avec des marquages (5) pour la saisie de l'outil de calibrage (C) par un système de navigation pour applications médicales, **caractérisé en ce qu'**au moins un des supports (4) peut être démonté de l'outil de calibrage (C).

2. Outil de calibrage suivant la revendication 1, **caractérisé en ce que** les structures de localisation sont des structures de billes ou de lignes reproductibles par rayonnement X, en particulier en tungstène.

3. Outil de calibrage suivant la revendication 1 ou 2, **caractérisé en ce qu'**il est disposé sur le capteur d'image de l'appareil à rayons X.

4. Outil de calibrage suivant l'une des revendications 1 à 3, **caractérisé en ce que** le support amovible (4) est disposé à l'aide d'une fermeture rapide sur le support non amovible (3).

5. Outil de calibrage suivant la revendication 4, **caractérisé en ce qu'**on utilise une fermeture à baïonnette ou un assemblage à emboîtement pour la fixation du support amovible (4).

6. Outil de calibrage suivant l'une des revendications 1 à 5, **caractérisé en ce que** les marquages (5) pour la saisie de l'outil de calibrage (C) par un système de navigation pour applications médicales sont disposés sur le support non amovible (3) ou sur le dispositif (2) de placement de l'outil de calibrage (C) dans le chemin de rayonnement de l'appareil à rayons X.

7. Outil de calibrage suivant l'une des revendications 1 à 5, **caractérisé en ce que** les marquages (5) sont disposés sur un support séparé, en particulier sur un film adhésif (20) de préférence détachable qui est appliqué sur l'appareil à rayons X ou sur le capteur d'image (1) de l'appareil à rayons X.
